# EUROPEAN PATENT SPECIFICATION

(11) **EP 2 247 952 B1**
(45) Date of publication and mention of the grant of the patent: **17.04.2013**
(21) Application number: 09716542.7
(22) Date of filing: 06.03.2009
(51) Int. Cl.: G01N 33/569, C07K 14/38

(54) **DIAGNOSIS OF AND VACCINATION AGAINST ASPERGILLUS FUMIGATUS**
DIAGNOSE VON UND IMPFUNG GEGEN ASPERGILLUS FUMIGATUS
DIAGNOSTIC DE ASPERGILLUS FUMIGATUS ET VACCINATION CONTRE CETTE INFECTION

(30) Priority: 06.03.2008 EP 08102363
(43) Date of publication of application: 10.11.2010
(73) Proprietor: Technische Universität Braunschweig, 38106 Braunschweig (DE)
(72) Inventor: SCHÜTTE, Mark, 38114 Braunschweig (DE); HUST, Michael, 30169 Hannover (DE); DÜBEL, Stefan, 38108 Braunschweig (DE)
(74) Representative: Taruttis, Stefan Georg
(86) International application number: PCT/EP2009/052691
(87) International publication number: WO 2009/109662

(56) References cited:
- WO-A-2004/067709
- CRAMERI R: "RECOMBINANT ASPERGILLUS FUMIGATUS ALLERGENS: FROM THE NUCLEOTIDE SEQUENCES TO CLINICAL APPLICATIONS" INTERNATIONAL ARCHIVES OF ALLERGY AND IMMUNOLOGY, XX, XX, vol. 115, no. 2, 1 January 1998 (1998-01-01), pages 99-114, XP009077171 ISSN: 1018-2438 -& DATABASE EMBL 10 January 2006 (2006-01-10), "Probable glycosidase crf1 precursor (EC 3.2.-.-) (Crh-like protein 1) (Allergen Asp f 9)." XP002485990 Database accession no. Q8J0P4
- STEVENS DAVID A: "Vaccinate against aspergillosis! A call to arms of the immune system" CLINICAL INFECTIOUS DISEASES, vol. 38, no. 8, 15 April 2004 (2004-04-15), pages 1131-1136, XP008106559 ISSN: 1058-4838
- FELDMESSER MARTA: "Prospects of vaccines for invasive aspergillosis" MEDICAL MYCOLOGY, vol. 43, no. 7, November 2005 (2005-11), pages 571-587, XP008106558 ISSN: 1369-3786
- ARROYO JAVIER ET AL: "The GPI-anchored gas and Crh families are fungal antigens" YEAST, vol. 24, no. 4, April 2007 (2007-04), pages 289-296, XP008093643 ISSN: 0749-503X

### SEQUENCE LISTING

<110> Technische Universität Braunschweig
<120> Diagnosis of and vaccination against Aspergillus *fumigatus*
<130> T1005PCT
<160> 8
<170> PatentIn version 3.4
<210> 1
   <211> 333
   <212> PRT
   <213> Aspergillus *fumigatus*
<220>
   <221> SIGNAL
   <222> (1)..(19)
<400> 1
   Met Tyr Phe Lys Tyr Thr Ala Ala Ala Leu Ala Ala Val Leu Pro Leu
   1 5 10 15
   Cys Ser Ala Gln Thr Trp Ser Lys Cys Asn Pro Leu Glu Lys Thr Cys
   20 25 30
   Pro Pro Asn Lys Gly Leu Ala Ala Ser Thr Tyr Thr Ala Asp Phe Thr
   35 40 45
   Ser Ala Ser Ala Leu Asp Gln Trp Glu Val Thr Ala Gly Lys Val Pro
   50 55 60
   Val Gly Pro Gln Gly Ala Glu Phe Thr Val Ala Lys Gln Gly Asp Ala
   65 70 75 80
   Pro Thr Ile Asp Thr Asp Phe Tyr Phe Phe Phe Gly Lys Ala Glu Val
   85 90 95
   Val Met Lys Ala Ala Pro Gly Thr Gly Val Val Ser Ser Ile Val Leu
   100 105 110
   Glu Ser Asp Asp Leu Asp Glu Val Asp Trp Glu Val Leu Gly Gly Asp
   115 120 125
   Thr Thr Gln Val Gln Thr Asn Tyr Phe Gly Lys Gly Asp Thr Thr Thr
   130 135 140
   Tyr Asp Arg Gly Thr Tyr Val Pro Val Ala Thr Pro Gln Glu Thr Phe
   145 150 155 160
   His Thr Tyr Thr Ile Asp Trp Thr Lys Asp Ala Val Thr Trp Ser Ile
   165 170 175
   Asp Gly Ala Val Val Arg Thr Leu Thr Tyr Asn Asp Ala Lys Gly Gly
   180 185 190
   Thr Arg Phe Pro Gln Thr Pro Met Arg Leu Arg Leu Gly Ser Trp Ala
   195 200 205
   Gly Gly Asp Pro Ser Asn Pro Lys Gly Thr Ile Glu Trp Ala Gly Gly
   210 215 220
   Leu Thr Asp Tyr Ser Ala Gly Pro Tyr Thr Met Tyr Val Lys Ser Val
   225 230 235 240
   Arg Ile Glu Asn Ala Asn Pro Ala Glu Ser Tyr Thr Tyr Ser Asp Asn
   245 250 255
   Ser Gly Ser Trp Gln Ser Ile Lys Phe Asp Gly Ser Val Asp Ile Ser
   260 265 270
   Ser Ser Ser Ser Val Thr Ser Ser Thr Thr Ser Thr Ala Ser Ser Ala
   275 280 285
   Ser Ser Thr Ser Ser Lys Thr Pro Ser Thr Ser Thr Leu Ala Thr Ser
   290 295 300
   Thr Lys Ala Thr Pro Thr Pro Ser Gly Thr Ser Ser Gly Ser Asn Ser
   305 310 315 320
   Ser Ser Ser Ala Glu Pro Thr Ala Ala Val Leu Ala Phe
   325 330
<210> 2
   <211> 999
   <212> DNA
   <213> Aspergillus *fumigatus*
<220>
   <221> sig_peptide
   <222> (1)..(57)
<400> 2
   atgtatttca agtacacagc agcagcccta gctgcggtgc tccctctttg ctctgcacag    60
   acttggtcaa agtgcaatcc ccttgagaag acctgcccgc ccaacaaggg tcttgctgca   120
   tccacttaca ccgccgactt cacctcagct tcagctttgg atcaatggga agtcactgca   180
   ggcaaagttc ccgttggccc acagggcgcc gagttcactg tcgctaagca aggcgacgca   240
   cctaccattg acaccgactt ctacttcttc ttcggaaagg ccgaagtggt gatgaaggcc   300
   gctcctggca caggtgttgt tagcagcatc gtcctggagt cggatgatct ggatgaggtt   360
   gactgggaag tattgggcgg tgacaccact caggttcaga caaactactt tggcaaagga   420
   gacaccacca catatgaccg aggcacttac gtgcccgttg ccactcctca ggagactttc   480
   cacacctaca ccatcgactg gaccaaggat gccgttacct ggtctattga cggtgcggtc   540
   gtgcgtacgc tcacgtacaa cgatgccaag ggtggcactc gttttcctca gactcctatg   600
   cgcctgagac ttggcagctg ggccggcggc gaccccagca accccaaggg caccatcgag   660
   tgggccggtg gcttgaccgg ctacagcgcg ggaccgtaca ccatgtacgt caagtccgtc   720
   cgtatcgaga acgccaaccc cgccgagtcc tacacctact cggacaactc tggctcttgg   780
   cagagcatca agttcgacgg ctccgtcgat atctcctcca gctcttccgt gacctcctcc   840
   accaccagca ccgccagctc cgccagctct acctcgagca agaccccttc cacctccacc   900
   ctggccactt ccaccaaggc gactcccacc ccgtctggaa ccagctccgg ctctaactcg   960
   agctccagcg cggaacctac cgctgccgtg ttggcattc                          999
<210> 3
   <211> 20
   <212> DNA
   <213> artificial
<220>
   <223> Seq ID No. 3
<400> 3 20
   agcagcccta gctgcggtgc 20
<210> 4
   <211> 19
   <212> DNA
   <213> artificial
<220>
   <223> Seq ID No. 4
<400> 4 19
   cgctgccgtg ttggcattc 19
<210> 5
   <211> 36
   <212> DNA
   <213> artificial
<220>
   <223> Seq ID No. 5
<400> 5
   atataagcta gcgcacagac ttggtcaaag tgcaat 36
<210> 6
   <211> 34
   <212> DNA
   <213> artificial
<220>
   <223> Seq ID No. 6
<400> 6
   tattatgcgg ccgcgtaccg ctgccgtgtt ggca 34
<210> 7
   <211> 35
   <212> DNA
   <213> artificial
<220>
<220>
   <223> Seq ID No. 7
<400> 7
   tattatgcgg ccgcgctcga ggtagagctg gcgga 35
<210> 8
   <211> 30
   <212> DNA
   <213> artificial
<220>
   <223> Seq ID No. 8
<400> 8
   tattatgcgg ccgcggggtg gcggaggctc 30

## Description

The present invention relates to an antigen specific for invasive aspergillosis and the use of the antigen for a diagnostic test as well as the use of the antigen as a vaccine. The antigen is highly specific for invasive aspergillosis and therefore the diagnostic process is capable of differentiating an infection by *Aspergillus fumigatus* from an infection by *Candida,* especially from *Candida albicans.* The vaccine against aspergillosis is also based on the immunogenic properties of the antigen. Both the diagnostic test for *Aspergillus fumigatus* and the vaccine eliciting an immune response inducing immunoprotection against *Aspergillus fumigatus* are specific for *Aspergillus fumigatus.*

### State of the art

Bozza et al. (Microbes and Infection 4 (2002) 1281-1290) show that the administration of a protein termed Aspf16 obtained from *Aspergillus* when used as a vaccine did not result in an effective immunoprotection against subsequent challenge with viable *Aspergillus* conidia in immunosuppressed mice. In contrast, administration of the immunogenic ODN 1668 (TCC-ATC-ACG-TTC-CTG-ATG-CT) sequence was necessary to obtain an immune response to Aspf16 to protect mice against invasive *Aspergillus* infection. The effect of increasing the immunoprotective properties of proteins by combination with the ODN sequence was also shown for different *Aspergillus* proteins. Prior to the experimental immune challenge with *Aspergillus* conidia, the immune system of the test animals was affected by administration of the general suppressant of cell growth cyclophosphamid.

Denikus et al. (Infection and Immunity (2005) 4704 - 4713) quotes Bozza et al. in respect of the immunoprotective potential of Aspf16 without mentioning that Bozza et al. only found immunoprotection when administering recombinant proteins in combination with the ODN sequence 1668, which is known from Lipford et al. (Eur. J. Immunol. 27 (1997) 3420-3426). Denikus et al. identified an immune response against Aspf16 in *Aspergillus* infected rabbits.

Boywer and Denning (2007) postulate that the sequence for the Aspf16 described by Bozza et al. is no naturally occurring protein but the consequence of a frame-shift or a sequencing error.

Publication Q8J0P4 gives the amino acid sequence of a 395 aa protein termed Crfl, which is indicated to be an allergen in humans.

Crameri in Int. Arch. Allergy Immunol. 115: 99-114 (1998) quotes that *Aspergillus fumigatus* contains major allergens rAsp f1 and rAsp f3, and minor allergen rAsp f9, without giving the amino acid sequences of the proteins.

Arroyo et al. in Yeast 24: 289-296 (2007) describe that Crf1 protein from *A. fumigatus* is specifically recognized by sera from patients with *Aspergillus* or *Candida* infections.

WO2004/067709 gives the computer-assisted analysis of the *A. fumigatus* genome, without indicating the specificity of the presence of a protein for invasive aspergillosis.

During the preparation of the present invention, it has been found that an mRNA sequence encoding a protein of the size of Aspf16 as given in Bozza et al. could not be identified in total mRNA isolated from biopsies of patients with proven invasive aspergillosis.

Stevens et al in Clinical Infectious Diseases, 1113-1136 (2004) give an overview of vaccination against aspergillosis.

Feldmesser, Medical Mycology 571-587 (2005) reviews vaccines against aspergillosis, and especially relates to Aspf16 being bound by IgE and IgG4 of allergic aspergillosis patients.

### Objects of the invention

In view of prior art publications, especially Bozza et al., it is an object of the present invention to provide a novel protein that can be used as an antigen for eliciting an immune response specific for *Aspergillus,* especially *Aspergillus fumigatus,* especially for use in humans having an immune system impaired by depletion of neutrophils.

Further, it is an object of the present invention to provide the use of a protein and of antibodies specific for that protein, respectively, in a test kit for diagnosis and a diagnostic method for detecting a invasive aspergillosis, especially by *Aspergillus fumigatus,* without interaction with *Candida* or antibody directed against Candida, especially *Candida albicans.*

The invention in its broadest form is defined by claims 1, 2, 7, 8, 16, 17, 20 and 21.
1. Pharmaceutical composition containing an immunogen for eliciting an immune response in an animal, which immune response is protective against invasive aspergillosis, characterized in that
   the immunogen is in the form of a protein or in the form of a nucleic acid sequence encoding a protein, the protein comprising an amino acid sequence corresponding to Seq.-ID No.1.
2. Pharmaceutical composition according to claim 1, characterized in that Seq ID No. 1 contains at least one amino acid exchange selected from the group of
   C instead of Y in position 43 of Seq ID No. 1,
   L instead of W in position 122 of Seq ID No. 1,
   I orK instead of V in position 124 of Seq 1 DNo. 1,
   H instead of Q in position 197 of Seq ID No. 1, and
   I instead of R in position 203 of Seq ID No. 1.
7. Process for a diagnostic assay for detection of invasive aspergillosis in an animal from an analytical sample obtained from the animal, characterized by comprising the step of detecting the presence of a nucleic acid sequence encoding a protein comprising an amino acid sequence corresponding to Seq.-ID No. 1, or detecting the presence of an antibody directed against a protein comprising an amino acid sequence corresponding to Seq.-IDNo. 1.
8. Process according to claim 7, characterized in that Seq ID No. 1 contains at least one amino acid exchange selected from the group of
   C instead of Y in position 43 of Seq ID No. 1,
   L instead of W in position 122 of Seq ID No. 1,
   V instead of T or K in position 124 of Seq ID No. 1,
   H instead of Q in position 197 of Seq ID No. 1, and
   I instead of R in position 203 of Seq ID No. 1.
16. Test kit for diagnosing invasive aspergillosis in a patient according to a process of one of the preceding claims, characterized by comprising a protein comprising or consisting of an amino acid sequence corresponding to Seq.-ID No. 1.
17. Test kit according to claim 16, characterized in that Seq ID No. I contains at least one amino acid exchange selected from the group of
   C instead of Y in position 43 of Seq ID No. 1,
   L instead of W in position 122 of Seq ID No. 1,
   V instead ofT or K in position 124 of Seq ID No. 1,
   H instead of Q in position 197 of Seq ID No. 1, and
   I instead of R in position 203 of Seq ID No. 1.
20. Use of a protein comprising or consisting of an amino acid sequence corresponding to Seq.-ID No. 1 for the production of a pharmaceutical composition for eliciting an immune response in an animal, which immune response is protective against invasive aspergillosis.
21. Use according to claim 20, characterized in that Seq ID No. I contains at least one amino acid exchange selected from the group of
   C instead of Y in position 43 of Seq ID No. 1,
   L instead of W in position 122 of Seq ID No. 1,
   I or K instead of V in position 124 of Seq ID No. 1,
   H instead of Q in position 197 of Seq ID No. 1, and
   I instead of R in position 203 of Seq ID No. 1.

Preferred embodiments are defined by dependent claims 3-6, 9-15, 18, 19, 22 and 23.

### General description of the invention

The invention achieves the above mentioned objects by the process and compositions as defined in the claims. The invention is based on the finding that a protein, presently termed Crf2 and having the amino acid sequence Seq ID No. 1, is an antigen that is specific for an invasive *Aspergillus* infection in animals, preferably at least in humans and in birds, and which protein elicits an immune response specifically directed against *Aspergillus,* especially against *Aspergillus fumigatus.* The specificity of the presence of a protein comprising or consisting of an amino acid corresponding to Seq ID No. 1 in one embodiment is used by detecting in a body sample antibody directed against the protein Crf2, and in another embodiment by detecting a nucleic acid sequence encoding a protein comprising or consisting of an amino acid corresponding to Seq ID No. 1, preferably by detecting a nucleic acid sequence comprising or being comprised in, preferably consisting of Seq ID No. 2, e.g. by using PCR. As the genomic nucleic acid region encoding Crf2 also encodes Crf1 (sequence published in Q8J0P4) and Crf2 is presumed to be a splicing variant of the same genomic region, the nucleic acid sequence specific for Crf2 is the mRNA encoding Crf2, preferably according to Seq ID No. 2. Accordingly, it is preferred that the analytical method comprises the steps of isolating mRNA from a sample, reversely transcribing the mRNA to generate the corresponding cDNA, and to specifically identify the nucleic acid sequence encoding Seq. DI No. 1, e.g. by PCR. In a process comprising the amplification of cDNA encoding Crf2, the differentiation from DNA amplification products originating from Crf1 or another possible splicing product of a gene encoding Crf2 can be made by using at least one primer which only binds to the cDNA originating from the mRNA encoding Crf2, e.g. a primer spanning a splicing site, and/or by size determination of the amplification product originating from mRNA encoding Crf2.

In order to comprise natural derivatives of protein comprising or consisting of an amino acid corresponding to Seq ID No. 1, derivatives of Seq ID No. I having at least one amino acid exchange are included in the invention. Exemplary amino acid exchanges of Seq ID No. 1 are given below. When analysing for nucleic acid sequences encoding a protein comprising or consisting of an amino acid corresponding to Seq ID No. 1, it is preferred to include single base exchanges from Seq ID No. 2 in order to comprise natural derivatives of Seq ID No. 2. Exemplary nucleic acid exchanges of Seq ID No. 2 are given below. As the protein comprising or consisting of an amino acid sequence corresponding to Seq ID No. 1 and, a nucleic acid sequence encoding such a protein, respectively, is specific for invasive aspergillosis and does not indicate presence or absence of a *Candida* infection, the processes of the invention can include the step of differentiating the presence of invasive aspergillosis from the presence of a *Candida* infection.

Accordingly, the diagnostic assay for detection of invasive aspergillosis in an animal from an analytical sample obtained from the animal comprises the step of detecting the presence of a nucleic acid sequence encoding a protein comprising or consisting of an amino acid sequence corresponding to Seq.-ID No. 1, and/or the step of detecting the presence of an antibody directed against a protein comprising or consisting of an amino acid sequence corresponding to Seq.-ID No. 1, wherein Seq ID No. 1 comprises natural derivatives having at least one amino acid exchange, e.g. as described herein below.

In accordance with the specificity of Crf2 for an invasive *Aspergillus* infection, Crf2 and antibody elicited by Crf2 in an infected animal can be used in a process for diagnosing an invasive *Aspergillus* infection. Accordingly, in a first aspect the present invention is directed to a method for diagnosis by specifically detecting in a biopsy, e.g. in bronchoalveolar lavage fluid (BAL) in serum of an animal, an immune reaction with Crf2. In this diagnostic method, Crf2, preferably obtained by heterologous expression in a micro-organism and purification from the expression host, can be provided, preferably in an immobilized state, e.g. bound to a carrier substrate, and contacted with a sample, e.g. serum obtained from an animal, preferably from a human or a bird, followed by removal of antibody not bound to Crf2 and, subsequently, detection of antibody bound to Crf2, which antibody reaction is a specific indicator for invasive aspergillosis.

It has been found that the detection of anti-Crf2 antibody in a sample obtained from an animal, e.g. from serum, using interaction with Crf2 in a diagnostic assay is highly specific for invasive *Aspergillus* infections, especially by *Aspergillus fumigatus,* without detecting an immune response elicited by *Candida,* especially by *Candida albicans.* Further, the diagnostic methods using Crf2 do not give a specific reaction with sera obtained from individuals being infected with asthmatic bronchopulmonary aspergillosis. Accordingly, Crf2 provides the basis for a specific immunological detection of an immune response in an animal, especially in humans for invasive aspergillosis, which does not give a cross reaction with immune responses elicited by an infection by *Candida albicans* or asthmatic bronchopulmonary aspergillosis, i.e. an immunoassay using Crf2 is highly specific for invasive aspergillosis by *Aspergillus fumigatus* without giving unspecific, i.e. false positive reactions with sera containing an immune response to *Candida albicans* or to bronchopulmonary aspergillosis.

When testing cross-reactivity of Crf2 with immune responses elicited by *Candida* infection and or bronchopulmonary aspergillosis, no reaction of the respective sera with Crf2 was found. Further, neither serum from Crf2-immunized nor from PBS-immunized mice showed any reaction with hyphae or spores from *Candida albicans.* Accordingly, Crf2 can be used in a diagnostic test kit and in a diagnostic method, respectively, for specifically detecting invasive aspergillosis, especially by *Aspergillus fumigatus,* without indicating a Candida infection, nor bronchopulmonary aspergillosis.

In the analytical process using the detection of nucleic acid sequences encoding a protein comprising or consisting of an amino acid corresponding to Seq ID No. 1 or of its derivatives, it is preferred to specifically amplify the nucleic acid sequence, preferably Seq ID No. 2, and to detect the presence of amplification products. Specific amplification of Seq ID No. 2 is obtainable using pairs of nucleic acid primers specific for Seq ID No. 2, e.g. Seq ID No. 3 and Seq ID No. 4. Detection of amplification can be by known methods including size separation of amplification products, monitoring of the amplification reaction by change of fluorescence detectable from fluorescence labels attached to at least one of the primers, and/or nested PCR using secondary specific amplification of a section of the amplification products obtained from the amplification products generated from the sample.

In another aspect, the present invention on the basis of the high specificity of Crf2 for invasive aspergillosis, especially caused by *Aspergillus fumigatus,* provides for a vaccine containing Crf2 in the form of the protein or in the form of a nucleic acid sequence encoding Crf2, preferably contained in a eucaryotic expression cassette, for specifically eliciting a protective immune response in animals against invasive aspergillosis, especially against invasive aspergillosis by *Aspergillus fumigatus.* It has been found that the immune protection that is elicited by administration of a vaccine composition comprising a protein having the epitopes contained in Seq. ID No. 1, is also effective in humans and birds with depletion of neutrophils, e.g. caused subsequent to administration of the vaccine composition of the invention. The maintenance of an effective immune protection originally elicited by the vaccine composition even under the condition of depletion of neutrophils subsequent to administration of the vaccine composition is surprising in view of Bozza et al., because the use of the general cell growth inhibitor cyclophosphamid essentially leaves existing neutrophils intact, which form an effective part in the immune response against invasive aspergillosis.

Accordingly, the present invention provides for the use of Crf2 for the production of a vaccine composition for providing immune protection against *Aspergillus,* especially *Aspergillus fumigatus* in an animal, preferably humans or birds, especially when the recipients subsequent to administration of the vaccine composition have an immune system impaired by depletion of neutrophils.

Administration of a vaccine containing Crf2 according to Seq ID No. 1 or containing a eucaryotic expression cassette, e.g. mammalian, human or for birds, the expression cassette containing a nucleic acid sequence encoding Crf2 of Seq ID No. 1 functionally arranged adjacent a promoter sequence in 5' and a terminator sequence in 3', administration of the vaccine to an animal, exemplified by mice, resulted in immunoprotection of at least 90, preferably at least 95, more preferably 100% of immunized animals. The immune response caused by immunization with Crf2 was demonstrated to provide an efficient immunoprotection against subsequent inoculation with viable *Aspergillus fumigatus* conidia, even when prior to the artificial *Aspergillus* infection the immune system was impaired by depletion of neutrophils. Accordingly, the present invention provides for the use of Crf2 protein having Seq ID No. 1 for the production of a vaccine composition for eliciting a protective immune response in an animal even if the animal subsequent to administration of Crf2 is depleted of neutrophils. The depletion of neutrophils, which are a predominant portion of the natural immune response against invasive aspergillosis, can be caused by immunosuppression due to medical treatment or due to disease.

In contrast to vaccines containing a peptide having the amino acid sequence of Aspfl6 as described in Bozza et al., it could be shown that a vaccine consisting of only Crf2 according to Seq ID No. 1 as the specific immunogen, upon immunization produced an efficient immune response against challenge of animals by a viable conidia of *Aspergillus fumigatus.* For protective immunization, Crf2 protein, preferably in combination with formulation agents and/or an adjuvant was used. Preferably, the pharmaceutical composition used as a vaccine did not contain ODN oligonucleotides as described in Bozza et al.

Comparison of detailed analyses of immunized animals in respect of the immune response elicited by a vaccine based on Crf2 according to Seq ID No. 1 as the immunogen compared to the immune response to a vaccine containing Aspf16 as the immunogen showed that the vaccine according to the invention produced a higher titer of antibody specific against *Aspergillus fumigatus* hyphae which are specific for invasive aspergillosis, as well as a higher titre of T-cells (e.g. Th1, Th2) directed against *Aspergillus fimugatus,* including hyphae, especially against *Aspergillus fumigatus* grown under conditions specific for invasive aspergillosis.

### Detailed description of the invention

The invention is now described in greater detail by examples with reference to the figures, wherein
- Figure 1 at Seq. ID No 1 gives the amino acid sequence of Crf2 and the cDNA sequence encoding Crf2 as Seq ID No. 2,
- Figure 2 shows the course of the immunization by Crf2, namely under A) a schematic overview of the course of immunization, immunosuppression and challenge with *Aspergillus* conidia, under B) a graphic representation of the number of immunized and immunosuppressed surviving mice, under C) a Western blot containing Crf2 detected with serum of mice taken during the course of the immunization, and under D) a Whisker plot representing ELISA results from sera taken over the course of the immunization and inoculation with *Aspergillus* conidia,
- Figure 3 shows micrographs of *A. fumigatus* and *Candida,* respectively, incubated with sera from Crf2 immunized animals (Crf2) and a mock immunized mouse (PBS) having survived infection with *A. fumigatus* at day 0, in immune fluorescence (left column each) and light microscopy (right column each), and
- Figure 4 shows an alignment of cDNA sequences of aspf16 (asp_f16), crf1 (crf), crf2 of the invention, and aspf9 (asp_f9).

### Example 1: Cloning of the coding sequence for Crf2 and production of Crf2 by heterologous expression

In an attempt to amplify the coding sequence for Aspf16 as described by Bozza et al., primers terminally arranged in the given aspf16 sequence were designed that would be suitable for amplification of the complete coding sequence for full-length Aspf16.

In contrast to Bozza et al., who isolated nucleic acids from an *in vitro* culture of *Aspergillus fumigatus,* the present inventors used cDNA produced by reverse transcription PCR of mRNA isolated from a biopsy obtained from a human patient with proven invasive aspergillosis. During the preparation of the present invention, no amplificate representing the full-length coding sequence of 1,300 bp of Aspf16 was found when using the primers designed to hybridize to terminal sections of the full length aspf16 sequence. However, two PCR products, one of 855 bp and one of 1008 bp were found, the first encoding Aspf9 and the second not corresponding to a known gene product. However, analysis of the second amplification product identified a coding sequence given as Seq ID No. 2, encoding a protein presently termed Crf2 (Seq ID No. 1), encoding a total of 333 amino acids, which essentially correspond to sections of Aspf16 and Crf1, respectively. From the partial similarity of the newly found Crf2 with Crf1, it could be concluded in retrospect that Crf2 is a novel splice variant of Crf1, which splice variant is specific for invasive aspergillosis in humans.

In the reverse transcription reaction, oligo dT primers (Invitrogen, Karlsruhe) were used. First strand cDNA was used as a template for nested PCR, followed by a second amplification using the PCR product of the first PCR reaction. In the sequences given, restriction sites for NheI and NotI are underlined. PCR products were restricted with NheI and NotI and cloned into the expression vector pET 21A (+) (Novagen, Darmstadt), giving an expression vector for Crf2.

For simplification of purification, a second expression vector encoding Crf2 with an added biotin acceptor domain (Cloutier et al., Mol. Immunol. 37, 1067-1077) was cloned by integrating a biotin acceptor domain (BAD) encoding sequence into the Xbal / Nhel site of the expression vector, effectively positioning the biotin acceptor domain encoding sequence in 5' to the Crf2 encoding sequence. Alternatively, labelling could be made by attaching an amino acid sequence for strep-tag, flag-tag, E-tag, S-tag, myc-tag or yol-tag to the N-terminus or C-terminus of the immunogenic Crf2 peptide.

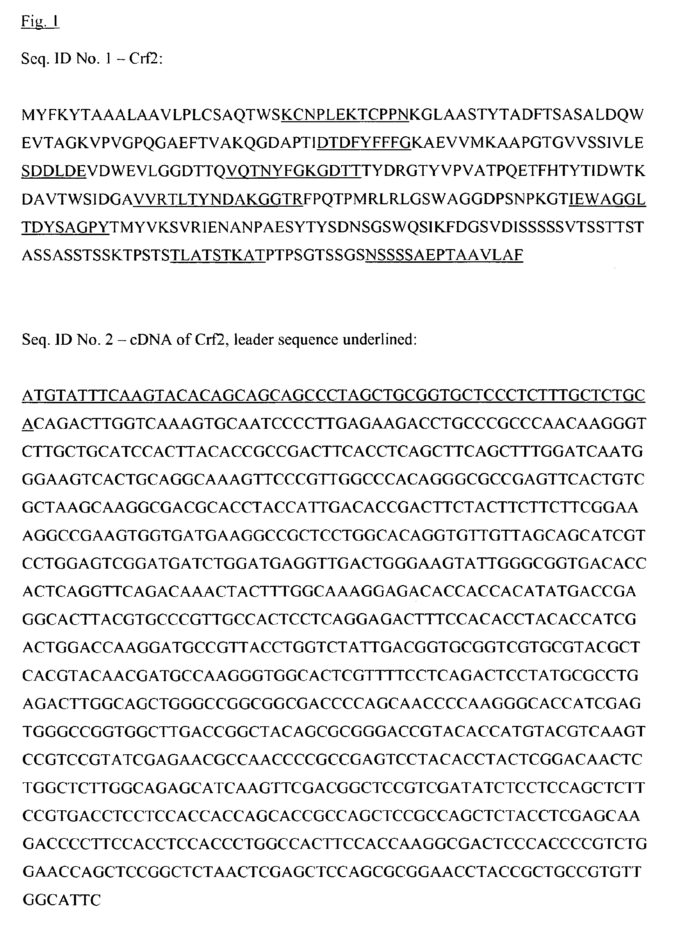

In Figure 1, the amino acid sequence of Crf2 according to the present invention is given as Seq. ID No 1, with the underlined sections being epitopes that have been identified by affinity of sera obtained from mice immunized with Crf2 according to Example 3. Due to the dense arrangement of epitopes in Crf2, for the purposes of the invention, the term Crf2 in addition to Seq. ID No 1 also extends to proteins of the same, longer or shorter length than Seq. ID No 1, which contain at least one, preferably 2, 3, 4, 5 or all 6 epitopes identified in Seq. ID No 1, with each epitope present independently in one or more copies.

For expression, XL1 - blue MRF' *E. coli* (Stratagene, Amsterdam) were transformed and grown in standard medium with added glucose and ampicillin and, in the case of the expression vector encoding Crf2 with an added biotin acceptor domain, including 50 µM biotin. After cultivation at 37 °C to an OD₆₀₀ <0.1, IPTG was added to 1 mM and cultivation was continued for 5 hours. Crf2 and Crf2 with an N-terminally added biotin acceptor domain could be isolated from harvested cells after cell disruption, e.g. by sonication. For purification of Crf2 in the case of labelled Crf2 containing an added biotin acceptor domain, affinity chromatography was used, followed by dialysis against 20 mM TrisHCl, pH 8 and, optionally, size exclusion chromatography and/or ion exchange chromatography.

When analysing biopsy material from different patients with proven invasive aspergillosis, it was found that the amino acid sequence of Crf2 could include at least one of the deviations from Seq.-ID No. 1 as listed in the table below, essentially without compromising the specificity of the Crf2 and of its natural variants for invasive aspergillosis nor the immunogenic properties to induce an immunoprotective response specifically directed against invasive aspergillosis.

**Table: amino acid deviations from Seq.-ID No. 1**

| amino acid position (numbering of Seq.-ID No.1) | amino acid in Seq.-ID No. 1 | amino acid exchange from Seq.-ID No. 1 |
|---|---|---|
| 43 | Y | C |
| 122 | W | L |
| 124 | V | I or K |
| 197 | Q | H |
| 203 | R | I |

### Example 2: Diagnosis of invasive aspergillosis by specific reaction with Crf2

Crf2 gives a highly specific immune reaction with sera from animals that had been exposed to invasive aspergillosis infection, but not with sera from animals suffering from bronchopulmonary aspergillosis or suffering from a *Candida* infection. In the diagnostic method of the invention, the high immune specificity of Crf2 to antibody elicited in animals in response to invasive aspergillosis, especially by *Aspergillus fumigatus* is utilized, which immune reaction does not occur with sera showing immune reactions specific for *Candida* infections or bronchopulmonary aspergillosis.

As an example for an ELISA for detecting the immune reactions of Crf2 with serum, recombinant purified Crf2, optionally containing a label, e.g. a biotin acceptor domain, was immobilized onto a substrate, e.g. onto a polystyrene microtiter plate. After blocking free binding sites with unspecific protein, the serum to be tested was added and incubated to allow for binding of antibody to immobilized Crf2. Following extensive washes to remove nonspecific or unbound antibody, bound antibody was detected, e.g. by a secondary antibody directed against the constant chain of the tested serum, which secondary antibody was coupled to a reporter dye or reporter enzyme.

Preferably, Crf2 without modification, namely consisting of the amino acid sequence of Seq ID No. 1 was immobilized to a carrier substrate followed by blocking and subsequent incubation with the serum to be tested, unbound antibody was removed. Specific interaction of serum antibody with bound Crf2 was by detection using incubation with labelled Crf2, with subsequent washing and specific detection of the label. For detection of the label, a suitable affinity detection or an indicator reaction using the generation of a detectable signal from added specific substrate was used. In this example, labelled Crf2 was Crf2 with an additional N-terminal biotin acceptor domain in combination with detection using a biotinylated reporter. Alternatively, labelled Crf2 was biotinylated Crf2, used in combination with subsequent detection of the biotin label, e.g. by incubation with streptavidin linked to a reporter dye or reporter enzyme, e.g. horse radish peroxidase followed by addition of a reporter substrate convertible by the reporter enzyme.

The diagnostic immunoassay using a first immune reaction of the serum to be tested with immobilized Crf2 with subsequent detection of bound antibody by reaction with labelled Crf2 had the advantage of high specificity while also detecting a large variety of immunoglobulins, including IgG, IgE and IgM.

When coating about 100 ng Crf2 produced according to Example 1 onto microtiter plates (Nunc Maxisorb, Nunc, Wiesbaden), followed by blocking with phosphate buffered saline including 0.1% (vol/vol) Tween20 and 2% (wt/vol) skim milk powder for 1.5 h, Crf2 was immobilized. Washing was with PBS, 0.1 % Tween20 without skim milk. Serum samples from experimentally infected mice or patients diagnosed with invasive aspergillosis were diluted about 1: 400 in PBS, Tween20, containing skim milk powder for 1 hour at room temperature. After washing, 100 ng biotinylated Crf2 was added to each well in PBS, Tween20 and incubated for 1 hour. After washing, detection was by incubation with streptavidin-conjugated to horse radish peroxidase, followed by washing and incubation with a substrate for horse radish peroxidase giving an optically detectable signal, e.g. using tetramethyl benzidine as the substrate.

As a result, it was found that only sera from mice or humans diagnosed with invasive aspergillosis yielded a signal in the immuno assay, whereas sera from humans or mice that were diagnosed with bronchopulmonary aspergillosis or a *Candida* infection but without invasive aspergillosis, did not give a positive result in the immunoassay using Crf2.

In the alternative to analysis of Crf2 protein, Crt2-like protein containing at least one amino acid exchange as given in the table above, and antibodies against such a Crf2-like protein was detected in clinical biopsies from patients with proven invasive aspergillosis.

As an alternative to the detection of Crf2 protein, of Crf2-like protein, or of antibody against one of these proteins in a biological sample, e.g. in an animal biopsy, nucleic acid sequences specific for theses proteins were detected, especially nucleic acid sequences corresponding to Seq ID No. 2 and the derivatives thereof as indicated herein.

In detail, nucleic acids were isolated from a patient sample, e.g. from blood or BAL, and used as a template to generate cDNA by reverse transcription (RT), e.g. using an oligo-dT primer and reverse transcriptase (RT reaction kit "SuperScript First-Strand", available from Invitrogen), and the cDNA was subjected to a PCR amplification. PCR conditions generally were 1 cycle 94°C, 60s, 30 cycles of 94°C, 30s, 60°C and 64°C, 30s, and 72°C, 150s, followed by 1 cycle of 72°C, 600s. PCR reactions were in 50µL containing 1.5 µL 50 mM MgCl₂, 5 µL 5x buffer, 1.5 µL of each primer, 1 µL dNTPs, 0.8 µL polymerase (Combizym), 2µL nucleic acid template, remainder water. For the PCR on the basis of cDNA generated from a sample using forward primary primer with a sequence corresponding to agc agc cct agc tgc ggt gc (Seq ID No. 3), and backward primary primer cgc tgc cgt gtt ggc att c (Seq ID No. 4). Amplification products were identified as Seq ID No. 2 by nested PCR using forward secondary primer ata taa gct agc gca cag act tgg tca aag tgc aat, containing a NheI site (Seq ID No. 5), and one of backward secondary primers tat tat gcg gcc gcg tac cgc tgc cgt gtt ggc a with a NotI site (Seq ID No. 6) or tat tat gcg gcg gcg ctc gag gta gag ctg gcg ga with a NotI site (Seq ID No. 7); recognition sites for restriction enzymes are underlined. Preferably, primer sequences are without nucleotides which are not complementary to Seq ID No. 2, e.g. without added recognition sites for restriction enzymes.

Interestingly, the only amplification product that was generated was for Crf2, but no amplificate of a size corresponding to crf1 or to aspf16 was found. This result indicates that the mRNA specifically coding for Crf2 could be identified by RT-PCR and, additionally, that the mRNAs specific for Crf1 or Aspf16 were not amplified in RT-PCR. This result is surprising as it is presently assumed that crf1 and aspf16 are transcription products of the same gene as crf2 (Seq ID No. 2).

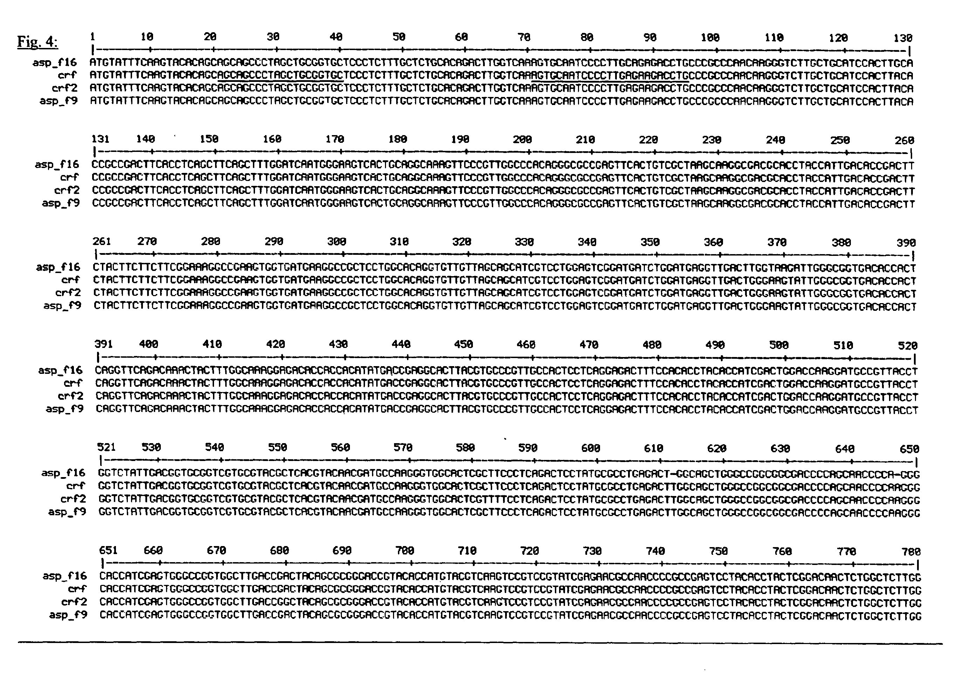

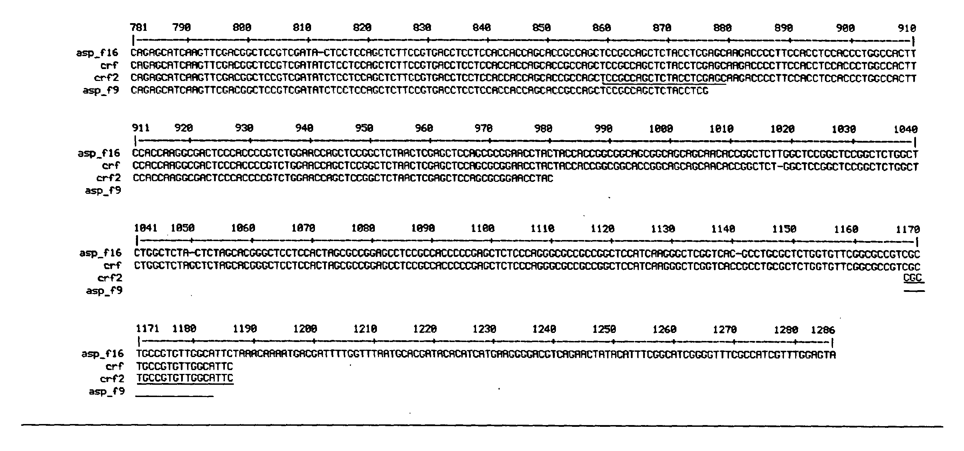

As a negative test, the analysis was repeated but replacing the secondary backward primer by tat tat gcg gcc gcg ggg tgg cgg agg ctc (Seq ID No. 8), which according to the genomic sequence hybridises to a nucleic acid section present in mRNA encoding Crfl and/or encoding Aspf16, which section is not present in mRNA encoding Crf2 (Seq ID No. 1). This PCR did not reveal a specific amplification product, demonstrating that the generation of an amplification product, e.g. by primers corresponding to Seq ID No 5 with one of Seq ID No 6 or 7 is specific for Seq ID No. 2 encoding Crf2 (Seq ID No. 1), and hence specific for the presence of an mRNA encoding Crf2, i.e. not comprising or extending to a splice variant such as Crf1 or Aspf16. In Figure 4, the primary primers (most 5' and most 3') and the secondary primers, which are arranged "nested", i.e. within a region embraced by the primary primers, are indicated by underlining.

The alignment of Figure 4 of putative splice variants shows the overlaps of the cDNA sequences encoding Aspf16 (asp_f16), Crf1 (crf), Crf2 according to the invention (crf2), and Aspf9 (asp_f9). It can be seen that the sequence encoding Crf2 (Seq ID No. 2) lacks nucleotides 980 to 1167 compared to crf1, and the sequence encoding Aspf16 in comparison to crf1 has deletions of nucleotides 611, 647, and 812, which deletions result in frame shift mutations.

### Example 3: Crf2 as a vaccine against invasive aspergillosis

The effective immunization against invasive aspergillosis using a vaccine containing Crf2 according to Seq ID No. 1 as the immunogen resulted in an efficient immunoprotection of mice against subsequent infection with conidia of *Aspergillus fumigatus,* which in mock-immunized mice regularly resulted in invasive aspergillosis infections.

C57 BL/6 (H-2b) mice were obtained from Harlan (Germany) and housed under pathogen free conditions. All experiments were approved by the animal protection committee of Braunschweig.

Mice were vaccinated once, followed by a second booster vaccination after 2 weeks, each subcutaneously into a neckfold using 50 µg Crf2 in an aqueous solution in 1:1 (vol/vol) admixture with an adjuvant (TiterMax TM, TiterMax Inc., USA) in a total volume of 60 µL. In the mock immunization, the solution of Crf2 was replaced by an equal volume of sterile PBS.

For the immuno assays, serum was obtained 1 hour before the first immunization, 1 hour before the artificial infection and directly before sacrificing infected mice. Blood was taken from the retro-orbital sinus and serum was separated by centrifugation and optionally frozen at -20 °C before testing using the ELISA.

In order to simulate the impaired function of the immune system in patients suffering from invasive aspergillosis, the experimental animals received 400 µg of RB6-8C5 antibody, which binds to the neutrophil specific antigen Gr-1 (Ly-6G (5)) for deleting neutrophils. Following this immunosuppression, immunized mice were put under a light anesthesia and were inoculated intranasally with 30 µL of a conidial suspension containing 5x10⁶ viable conidia while being held vertically. During recovery from anesthesia, mice were placed on their backs.

1-2 hours following anesthesia and inoculation, all animals recovered and had a normal appearance until first signs of invasive aspergillosis appeared 24-48 hours following infection. For avoiding unnecessary suffering, e.g. laboured breathing, a more than 20% weight loss and occurring specific motile disorders like a heavy rotation along the head - tail - axis, infected animals were killed. Prematurely killed animals were counted as having died from invasive aspergillosis, as was confirmed by later analysis.

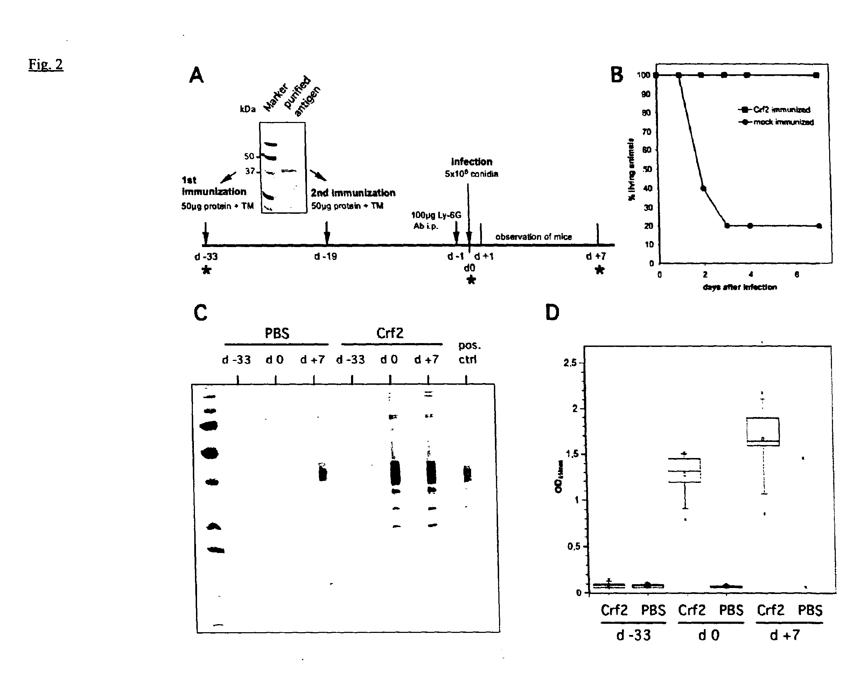

Figure 2 A) schematically shows the course of the immunization, consisting of a first immunization with purified Crf2 as the antigen at day -33, followed by a second booster immunization with purified Crf2 at day -19. One day before infection, immunosuppression was caused by intraperitoneal injection of the anti-neutrophil antibody. At day 0, artificial infection of the immunosuppressed animals was performed and mice were observed for the following 7 days.

In Figure 2 B), the number of surviving animals is shown, demonstrating that all the animals immunized with Crf2 survived subsequent to neutrophil depletion and nasal infection with *Aspergillus fumigatus* viable spores, proving that Crf2 is a potent immunogen for a vaccine for providing immunoprotection that is sufficient for protection against later infection by *Aspergillus* even under the conditions of immunosuppression following the immunization with Crf2.

Figure 2 C) shows a Western blot, wherein an SDS-PAGE was loaded with marker protein (left hand lane), 6 lanes loaded with an *E*. *coli* extract from recombinant *E*. *coli* expressing Crf2 of Seq ID No. 1, and affinity - purified labelled Crf2, carrying an N-terminal poly-histidine tag (right hand lane).

For detection, serum of one mock-immunized mouse (PBS) was used on separate strips of the Western blot membrane, namely serum taken at day -33, serum taken at day 0 and serum taken at day 7 following artificial infection. All sera were diluted 1:150. The His-tag labelled Crf2 serving as the positive control (pos. ctrl) was separately detected using an anti-His-tag antibody. The reaction of the serum from the mock-immunized mouse at day +7 indicates that this animal had also developed antibody specifically directed against Crf2.

The analysis of the serum from the later Crf2-immunized mouse shows that at day -37, that is prior to immunization, no antibody specific for Crf2 is detected, whereas at day 0, e.g. following the first immunization and the booster immunization with Crf2, a strong antibody reaction has developed, which is detectable through day 7 after the artificial infection with *Aspergillus* conidia.

The immune response at day -33, day 0 and day 7 for the Crf2-immunized mouse serum and the mock-immunized mouse serum used for Figure 2 C) was analysed in the preferred in ELISA using immobilized Crf2, contacting with serum, followed by detection using labelled Crf2, optionally followed by an indicator reaction caused by the label. The results are shown in the Whisker plot of Figure 2 D), showing the negligible background reaction to Crf2 at day -33 for both the later Crf2-immunized and the mock-immunized (PBS) mouse sera. Following the two - staged immunization using Crf2, the Crf2-immunized mouse shows a drastically augmented Crf2-specific immune response, whereas the mock-immunized mouse only shows the original background reaction against Crf2. Following challenge with viable *Aspergillus fumigatus* viable spores, the Crf2-immunized mouse even shows a further increase in the immune response to Crf2, whereas the mock-immunized mouse only at this stage shows an induced antibody reaction.

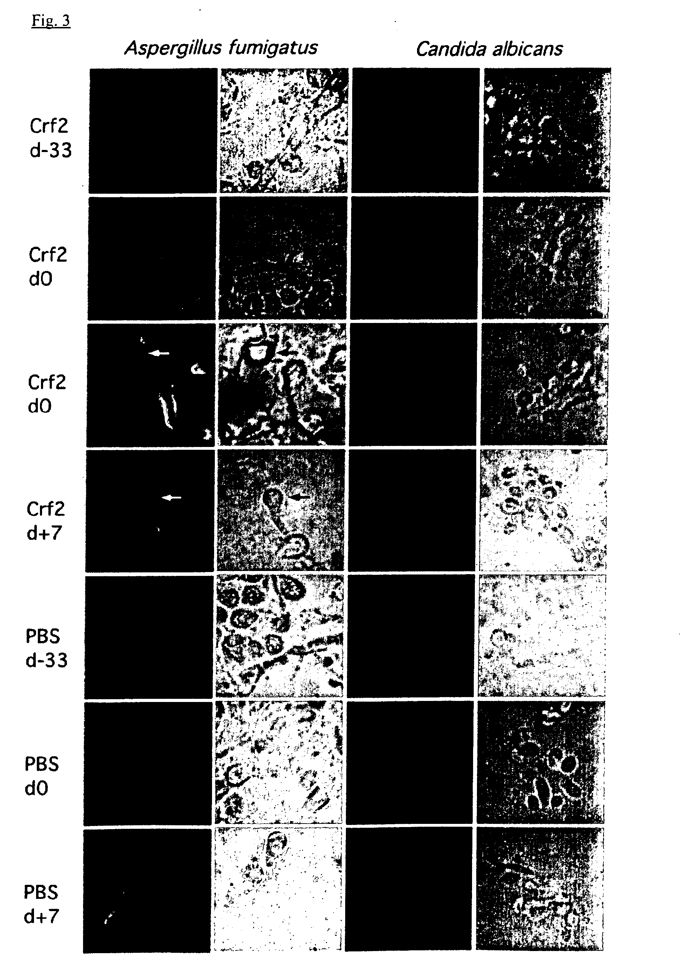

Figure 3 shows the reaction of sera obtained from the experimental animals in row 1 at day - 33 (d-33), just prior to vaccination with Crf2, at day 0 (d0), i.e. 33 days after vaccination with Cry2., in rows 2 and 3. At day 0, animals were infected with *Aspergillus* conidia and, accordingly, day +7 (d+7) shows the reaction of sera following artificial infection. Generally, in Figure 3, the left column of pictures *Aspergillus fumigatus* and *Candida,* respectively shows immune fluorescence micrographs of sera incubated with *Aspergillus fumigatus* and *Candida* cultivated in RPMI 1640-FCS medium (obtained from PAA, Cölbe, Germany) for 24 h on microscope slides. After cultivation, medium was removed, slides were blocked with blocking buffer (M-PBS, Tween20, 3% BSA) for 1.5 h, then washed with PBS. Following incubation with diluted sera and washing, detection of bound antibody was with goat antimouse (Fc specific), conjugated with Alexa 488 (Invitrogen, Germany), washing with water and observed under a Zeiss Axiovert 200 (Zeiss Jena, Germany) fluorescence microscope. The right column of pictures shows the respective light micrographs.

It can be seen that neither cultivated *Aspergillus fumigatus* hyphae nor *Candida* are recognized by sera prior to Crf2 immunization. Following Crf2 immunization, sera at day 0 recognize *Aspergillus* hyphae but not *Candida.* From the results of the immuno assays, it is concluded that in hyphae, Crf2 protein is detected by sera. When comparing the fluorescence with the light micrograph, it can be seen that sera predominantly react with the hyphae but not with the spore (indicated by arrow), indicating that immunization with Crf2 elicits a protective immune response against actively growing *Aspergillus* cells.

Further, sera taken subsequent to artificial infection (d+7) detect *Aspergillus* hyphae but again not *Candida.* Sera from the mock (PBS) immunized mouse that survived artificial infection only at day 7 (d+7) reacts with *Aspergillus* hyphae, indicating that Crf2 also in infection without prior immunization is the target of the immune response, making Crf2 a suitable vaccine against invasive aspergillosis.

As well as sera from Crf2 immunized animals, sera from the mock immunized mouse neither before nor subsequent to artificial *Aspergillus* infection reacts with *Candida,* proving that Crf2 and the antibodies specific for Crf2 are suitable reactants for specific diagnosis of invasive aspergillosis with differentiation from *Candida* infection, i.e. without reacting with *Candida.*

## Claims

1. Pharmaceutical composition containing an immunogen for eliciting an immune response in an animal, which immune response is protective against invasive aspergillosis, **characterized in that** the immunogen is in the form of a protein or in the form of a nucleic acid sequence encoding a protein, the protein comprising an amino acid sequence corresponding to Seq.-ID No. 1.

2. Pharmaceutical composition according to claim 1, **characterized in that** Seq ID No. 1 contains at least one amino acid exchange selected from the group of
C instead of Y in position 43 of Seq ID No. 1,
L instead of W in position 122 of Seq ID No. 1,
I or K instead of V in position 124 of Seq ID No. 1,
H instead of Q in position 197 of Seq ID No. 1, and
I instead of R in position 203 of Seq ID No. 1.

3. Pharmaceutical composition according to one of the preceding claims, **characterized in that** at least following administration of the pharmaceutical composition, the animal has an impaired immune system.

4. Pharmaceutical composition according to claim 3, **characterized in that** the impairment of the immune system is caused by an HIV infection or by the administration of immunosuppressive compounds.

5. Pharmaceutical composition according to one of claims 3 or 4, **characterized in that** the impairment of the immune system is the depletion of neutrophils.

6. Pharmaceutical composition according to one of the preceding claims, **characterized in that** the animal is a human or a bird.

7. Process for a diagnostic assay for detection of invasive aspergillosis in an animal from an analytical sample obtained from the animal, **characterized by** comprising the step of detecting the presence of a nucleic acid sequence encoding a protein comprising an amino acid sequence corresponding to Seq.-ID No. 1, or detecting the presence of an antibody directed against a protein comprising an amino acid sequence corresponding to Seq.-ID No. 1.

8. Process according to claim 7, **characterized in that** Seq ID No. 1 contains at least one amino acid exchange selected from the group of
C instead of Y in position 43 of Seq ID No. 1,
L instead of W in position 122 of Seq ID No. 1,
V instead of I or K in position 124 of Seq ID No. 1,
H instead of Q in position 197 of Seq ID No. 1, and
I instead of R in position 203 of Seq ID No. 1.

9. Process according to claim 7 or 8, **characterized by** comprising the steps of contacting a protein comprising or consisting of an amino acid sequence corresponding to Seq.-ID No. 1 with the analytical sample, and identifying binding of antibody of the analytical sample to the protein comprising or consisting of an amino acid sequence corresponding to Seq.-ID No. 1.

10. Process according to one of claim 9, **characterized in that** subsequent to contacting the analytical sample with the protein comprising or consisting of an amino acid sequence corresponding to Seq.-ID No. 1, the analytical sample is contacted with a labelled protein comprising or consisting of an amino acid sequence corresponding to Seq.-ID No. 1, followed by detection of the label.

11. Process according to claim 10, **characterized in that** the label is selected from a poly-histidine sequence, a biotin acceptor domain, biotinylation, a strep-tag, flag-tag, E-tag, S-tag, myc-tag and yol-tag.

12. Process according to one of claims 8 to 11, **characterized by** its use for differentiation of invasive aspergillosis from bronchopulmonary aspergillosis and/or from an infection by *Candida.*

13. Process according to claim 7, **characterized by** comprising the steps of isolating nucleic acids from the analytical sample and identifying a nucleic acid sequence comprising or consisting of a nucleic acid sequence corresponding to Seq ID No. 2.

14. Process according to claim 13, **characterized in that** isolating nucleic acids contains the step of isolating mRNA, and the step of identifying a nucleic acid sequence comprising or consisting of a nucleic acid sequence corresponding to Seq ID No. 2 comprises the step of reversely transcribing the mRNA and subjecting the mRNA to a first PCR using a pair of primary primers which correspond to and/or which are complementary to sections of Seq ID No. 2.

15. Process according to one of claims 14 or 15, **characterized in that** the amplification products of the first PCR are subject to a secondary PCR using primers that hybridise to Seq ID No. 2 in sections which do not correspond to and which are not complementary to the sequences of the primary primers.

16. Test kit for diagnosing invasive aspergillosis in a patient according to a process of one of the preceding claims, **characterized by** comprising a protein comprising or consisting of an amino acid sequence corresponding to Seq.-ID No. 1.

17. Test kit according to claim 16, **characterized in that** Seq ID No. 1 contains at least one amino acid exchange selected from the group of
C instead of Y in position 43 of Seq ID No. 1,
L instead of W in position 122 of Seq ID No. 1,
V instead of I or K in position 124 of Seq ID No. 1,
H instead of Q in position 197 of Seq ID No. 1, and
I instead of R in position 203 of Seq ID No. 1.

18. Test kit according to claim 16 or 17, **characterized in that** the protein in addition to amino acid sequence Seq ID No. 1 contains at least one detectable label.

19. Test kit according to one of claims 16 to 18, **characterized in that** the label is selected from biotinylation, a poly-histidine sequence, a biotin acceptor domain, strep-tag, flag-tag, E-tag, S-tag, myc-tag and yol-tag.

20. Use of a protein comprising or consisting of an amino acid sequence corresponding to Seq.-ID No. 1 for the production of a pharmaceutical composition for eliciting an immune response in an animal, which immune response is protective against invasive aspergillosis.

21. Use according to claim 20, **characterized in that** Seq ID No. 1 contains at least one amino acid exchange selected from the group of
C instead of Y in position 43 of Seq ID No. 1,
L instead of W in position 122 of Seq ID No. 1,
I or K instead of V in position 124 of Seq ID No. 1,
H instead of Q in position 197 of Seq ID No. 1, and
I instead of R in position 203 of Seq ID No. 1.

22. Use according to claim 20 or 21, **characterized in that** the animal has an impaired immune system.

23. Use according to claim 22, **characterized in that** the impairment of the immune system includes the depletion of neutrophils.

## Patentansprüche

1. Pharmazeutische Zusammensetzung mit einem Immunogen zum Auslösen einer Immunantwort in einem Lebewesen, die gegen invasive Aspergillose schützt, **dadurch gekennzeichnet, dass**
das Immunogen in Form eines Proteins oder in Form einer Nukleinsäuresequenz ist, die ein Protein kodiert, wobei das Protein eine Aminosäuresequenz gemäß Seq.- ID Nr. 1 umfasst.

2. Pharmazeutische Zusammensetzung nach Anspruch 1, **dadurch gekennzeichnet, dass** Seq.- ID Nr. 1 zumindest einen Aminosäureaustausch enthält, der ausgewählt ist aus der Gruppe von
C anstelle von Y an Position 43 von Seq.- ID Nr. 1,
L anstelle von W an Position 122 von Seq.- ID Nr. 1,
I oder K anstelle von V an Position 124 von Seq.- ID Nr. 1,
H anstelle von Q an Position 197 von Seq.- ID Nr. 1, und
I anstelle von R an Position 203 von Seq.- ID Nr. 1.

3. Pharmazeutische Zusammensetzung nach einem der voranstehenden Ansprüche, **dadurch gekennzeichnet, dass** das Lebewesen zumindest nach der Verabreichung der pharmazeutischen Zusammensetzung ein beeinträchtigtes Immunsystem hat.

4. Pharmazeutische Zusammensetzung nach Anspruch 3, **dadurch gekennzeichnet, dass** die Beeinträchtigung des Immunsystems durch eine HIV-Infektion oder durch die Verabreichung von immunsuppressiven Verbindungen verursacht ist.

5. Pharmazeutische Zusammensetzung nach einem der Ansprüche 3 oder 4, **dadurch gekennzeichnet, dass** die Beeinträchtigung des Immunsystems die Depletion neutrophiler Leukozyten ist.

6. Pharmazeutische Zusammensetzung nach einem der voranstehenden Ansprüche, **dadurch gekennzeichnet, dass** das Lebewesen ein Mensch oder ein Vogel ist.

7. Diagnostisches Analyseverfahren zur Detektion von invasiver Aspergillose in einem Lebewesen anhand einer von dem Lebewesen erhaltenen analytischen Probe, **dadurch gekennzeichnet, dass** es den Schritt des Detektierens der Anwesenheit einer Nukleinsäuresequenz, die ein Protein kodiert, das eine Aminosäuresequenz gemäß Seq.- ID Nr. 1 aufweist, oder des Detektierens der Anwesenheit eines Antikörpers, der gegen ein Protein gerichtet ist, das eine Aminosäuresequenz gemäß Seq.- ID Nr. 1 aufweist, umfasst.

8. Verfahren nach Anspruch 7, **dadurch gekennzeichnet, dass** Seq.- ID Nr. 1 zumindest einen Aminosäureaustausch enthält, der ausgewählt ist aus der Gruppe von
C anstelle von Y an Position 43 von Seq.- ID Nr. 1,
L anstelle von W an Position 122 von Seq.- ID Nr. 1,
V anstelle von I oder K an Position 124 von Seq.- ID Nr. 1,
H anstelle von Q an Position 197 von Seq.- ID Nr. 1, und
I anstelle von R an Position 203 von Seq.- ID Nr. 1.

9. Verfahren nach Anspruch 7 oder 8, **dadurch gekennzeichnet, dass** es die Schritte des Kontaktierens eines Proteins, das eine Aminosäuresequenz gemäß Seq.- ID Nr. 1 umfasst oder daraus besteht, mit der analytischen Probe, und des Identifizierens des Bindens eines Antikörpers der analytischen Probe an das Protein umfasst, das eine Aminosäuresequenz gemäß Seq.- ID Nr. 1 umfasst oder daraus besteht.

10. Verfahren nach Anspruch 9, **dadurch gekennzeichnet, dass** die analytische Probe nach dem Kontaktieren der analytischen Probe mit dem Protein, das eine Aminosäuresequenz gemäß Seq.- ID Nr. 1 umfasst oder daraus besteht, mit einem markierten Protein kontaktiert wird, das eine Aminosäuresequenz gemäß Seq.- ID Nr. 1 aufweist oder daraus besteht, gefolgt von der Detektion der Markierung.

11. Verfahren nach Anspruch 10, **dadurch gekennzeichnet, dass** die Markierung ausgewählt ist unter einer Polyhistidin-Sequenz, einer Biotinakzeptordomäne, einer Biotinylierung, einer strep-Markierung, einer flag-Markierung, einer E-Markierung, einer S-Markierung, einer myc-Markierung und einer yol-Markierung.

12. Verfahren nach einem der Ansprüche 8 bis 11, **gekennzeichnet durch** dessen Verwendung zur Unterscheidung von invasiver Aspergillose von bronchopulmonaler Aspergillose und/oder von einer Infektion **durch** *Candida.*

13. Verfahren nach Anspruch 7, **dadurch gekennzeichnet, dass** es die Schritte des Isolierens von Nukleinsäuren aus der analytischen Probe und des Identifizierens einer Nukleinsäuresequenz, die eine Nukleinsäuresequenz gemäß Seq.- ID Nr. 2 umfasst oder daraus besteht, umfasst.

14. Verfahren nach Anspruch 13, **dadurch gekennzeichnet, dass** das Isolieren der Nukleinsäuren den Schritt des Isolierens von mRNA enthält und der Schritt des Identifizierens einer Nukleinsäuresequenz, die eine Nukleinsäuresequenz gemäß Seq.-ID Nr. 2 umfasst oder daraus besteht, den Schritt des reversen Transkribierens der mRNA und des Unterziehens der mRNA einer ersten PCR unter Verwendung eines Paares von Primärprimem umfasst, die gemäß und/oder komplementär zu Abschnitten von Seq.- ID Nr. 2 sind.

15. Verfahren nach einem der Ansprüche 14 oder 15, **dadurch gekennzeichnet, dass** die Amplifikationsprodukte der ersten PCR Gegenstand einer zweiten PCR unter Verwendung von Primem sind, die mit Seq.- ID Nr. 2 in Abschnitten hybridisieren, die nicht gemäß und nicht komplementär zu den Sequenzen der Primärprimer sind.

16. Testkit zum Diagnostizieren von invasiver Aspergillose in einem Patienten nach einem Verfahren der voranstehenden Ansprüche, **dadurch gekennzeichnet, dass** es ein Protein umfasst, das eine Aminosäuresequenz gemäß Seq.- ID Nr. 1 umfasst oder daraus besteht.

17. Testkit nach Anspruch 16, **dadurch gekennzeichnet, dass** Seq.- ID Nr. 1 zumindest einen Aminosäureaustausch enthält, der ausgewählt ist aus der Gruppe von
C anstelle von Y an Position 43 von Seq.- ID Nr. 1,
L anstelle von W an Position 122 von Seq.- ID Nr. 1,
V anstelle von I oder K an Position 124 von Seq.- ID Nr. 1,
H anstelle von Q an Position 197 von Seq.- ID Nr. 1, und
I anstelle von R an Position 203 von Seq.- ID Nr. 1.

18. Testkit nach einem der Ansprüche 16 oder 17, **dadurch gekennzeichnet, dass** das Protein zusätzlich zu der Aminosäuresequenz Seq.- ID Nr. 1 zumindest eine detektierbare Markierung enthält.

19. Testkit nach einem der Ansprüche 16 bis 18, **dadurch gekennzeichnet, dass** die Markierung ausgewählt ist unter Biotinylierung, einer Polyhistidin-Sequenz, einer Biotinakzeptordomäne, einer strep-Markierung, einer flag-Markierung, einer E-Markierung, einer S-Markierung, einer myc-Markierung und einer yol-Markierung.

20. Verwendung eines Proteins, das eine Aminosäuresequenz gemäß Seq.- ID Nr. 1 umfasst oder daraus besteht, zur Herstellung einer pharmazeutischen Zusammensetzung zum Auslösen einer Immunantwort in einem Lebewesen, die gegen invasive Aspergillose schützt.

21. Verwendung nach Anspruch 20, **dadurch gekennzeichnet, dass** Seq.- ID Nr. 1 zumindest einen Aminosäureaustausch enthält, der ausgewählt ist aus der Gruppe von
C anstelle von Y an Position 43 von Seq.- ID Nr. 1,
L anstelle von W an Position 122 von Seq.- ID Nr. 1,
I oder K anstelle von V an Position 124 von Seq.- ID Nr. 1,
H anstelle von Q an Position 197 von Seq.- ID Nr. 1, und
I anstelle von R an Position 203 von Seq.- ID Nr. 1.

22. Verwendung nach Anspruch 20 oder 21, **dadurch gekennzeichnet, dass** das Lebewesen ein beeinträchtigtes Immunsystem hat.

23. Verwendung nach Anspruch 22, **dadurch gekennzeichnet, dass** die Beeinträchtigung des Immunsystems die Depletion von neutrophilen Leukozyten einschließt.

## Revendications

1. Composition pharmaceutique contenant un immunogène pour susciter une réponse immunitaire chez l'animal, laquelle réponse immunitaire protège contre l'aspergillose envahissante, **caractérisée en ce que**
l'immunogène se présente sous la forme d'une protéine ou sous forme d'une séquence d'acide nucléique de codage d'une protéine, la protéine comprenant une séquence d'acides aminés correspondant à la séquence identifiée no 1.

2. Composition pharmaceutique selon la revendication 1, **caractérisée en ce que** la séquence identifiée no 1 contient au moins un échange d'acide aminé sélectionné parmi le groupe de
C au lieu de Y en position 43 de la séquence identifiée no 1,
L au lieu de W en position 122 de la séquence identifiée no 1,
I ou K au lieu de V en position 124 de la séquence identifiée no 1,
H au lieu de Q en position 197 de la séquence identifiée no 1,
et I au lieu de R en position 203 de la séquence identifiée no 1.

3. Composition pharmaceutique selon l'une des revendications précédentes, **caractérisée en ce qu'**au moins après l'administration de la composition pharmaceutique, l'animal présente un système immunitaire déficient.

4. Composition pharmaceutique selon la revendication 3, **caractérisée en ce que** la déficience du système immunitaire est provoquée par une infection HIV ou par l'administration de composés immunosuppresseurs.

5. Composition pharmaceutique selon l'une des revendications 3 ou 4, **caractérisée en ce que** la déficience du système immunitaire est l'épuisement des neutrophiles.

6. Composition pharmaceutique selon l'une des revendications précédentes, **caractérisée en ce que** l'animal est un humain ou un oiseau.

7. Méthode d'essai diagnostique de détection de l'aspergillose envahissante chez un animal à partir d'un prélèvement analytique obtenu à partir de l'animal, **caractérisée en ce qu'**elle comprend la phase de détection de la présence d'une séquence d'acide nucléique de codage d'une protéine comprenant une séquence d'acides aminés correspondant à la séquence identifiée no 1. ou de détection de la présence d'un anticorps dirigé vers une protéine comprenant une séquence d'acides aminés correspondant à la séquence identifiée no 1.

8. Procédé selon la revendication 7, **caractérisée en ce que** la séquence identifiée no 1 contient au moins un échange d'acide aminé sélectionné parmi le groupe de
C au lieu de Y en position 43 de la séquence identifiée no 1,
L au lieu de W en position 122 de la séquence identifiée no 1,
V au lieu de I ou K en position 124 de la séquence identifiée no 1,
H au lieu de Q en position 197 de la séquence identifiée no 1,
et I au lieu de R en position 203 de la séquence identifiée no 1.

9. Procédé selon la revendication 7 ou 8, **caractérisé en ce qu'**il comprend les phases de contact avec une protéine comprenant ou consistant en une séquence d'acides aminés correspondant à la séquence identifiée no 1 avec le prélèvement analytique, et d'identification de la liaison d'anticorps du prélèvement analytique avec la protéine comprenant ou consistant en une séquence d'acides aminés correspondant à la séquence identifiée no 1.

10. Procédé selon la revendication 9, **caractérisé en ce qu'**après le contact du prélèvement analytique avec la protéine comprenant ou consistant en une séquence d'acides aminés correspondant à la séquence identifiée no 1, le prélèvement analytique est mis en contact avec une protéine marquée comprenant ou consistant en une séquence d'acides aminés correspondant à la séquence identifiée no 1, suivie de la détection de l'étiquette.

11. Procédé selon la revendication 10, **caractérisé en ce que** l'étiquette est sélectionnée parmi une séquence polyhistidine, un domaine accepteur de biotine, biotinylation, une étiquette Strep, une étiquette Flag, une étiquette E, une étiquette S, une étiquette myc et une étiquette yol.

12. Procédé selon l'une des revendications 8 à 11, **caractérisé par** son utilisation pour la différenciation de l'aspergillose envahissante de l'aspergillose bronchopulmonaire et/ou d'une infection par *Candida.*

13. Procédé selon la revendication 7, **caractérisé en ce qu'**il comprend les phases d'isolation des acides nucléiques du prélèvement analytique et d'identification d'une séquence d'acide nucléique comprenant ou consistant en une séquence d'acide nucléique correspondant à une séquence identifiée no 2.

14. Procédé selon la revendication 13, **caractérisé en ce que** l'isolation des acides nucléiques contient la phase d'isolation de mARN et la phase d'identification d'une séquence d'acide nucléique comprenant ou consistant en une séquence d'acide nucléique correspondant à la séquence identifiée no 1 comprend la phase de transcription inverse du mARN et du soumission du mARN à un premier PCR à l'aide d'une paire d'amorces primaires qui correspondent à et/ou qui sont complémentaires de sections de la séquence identifiée no 2.

15. Procédé selon l'une des revendications 14 à 15, **caractérisé en ce que** les produits d'amplification du premier PCR sont soumis à un PCR secondaire à l'aide d'amroces s'hybridant en séquence identifiée no 2 en sections qui ne correspondent pas à et qui ne sont pas complémentaires des séquences des amorces primaires.

16. Kit test de diagnostic de l'aspergillose envahissante chez un patient selon un procédé de l'une des revendications précédentes, **caractérisé en ce qu'**il comprend une protéine comprenant ou consistant en une séquence d'acides aminés correspondant à la séquence identifiée no 1.

17. Kit test selon la revendication 16, **caractérisé en ce que** la séquence identifiée no 1 contient au moins un échange d'acide aminé sélectionné parmi le groupe de
C au lieu de Y en position 43 de la séquence identifiée no 1,
L au lieu de W en position 122 de la séquence identifiée no 1,
V au lieu de I ou K en position 124 de la séquence identifiée no 1,
H au lieu de Q en position 197 de la séquence identifiée no 1,
et I au lieu de R en position 203 de la séquence identifiée no 1.

18. Kit test selon la revendication 16 ou 17, **caractérisé en ce que** la protéine, outre la séquence d'acides aminés, de séquence identifiée no 1 contient au moins une étiquette détectable.

19. Kit test selon l'une des revendications 16 ou 18, **caractérisé en ce que** l'étiquette est sélectionnée parmi la biotinylation, une séquence polyhistidine, un domaine accepteur de biotine, une étiquette Strep, une étiquette Flag, une étiquette E, une étiquette S, une étiquette myc et une étiquette yol.

20. Utilisation d'une protéine comprenant ou consistant en une séquence d'acides aminés correspondant à la séquence identifiée no 1 pour la production d'une composition pharmaceutique pour déclencher une réponse immunitaire chez un animal, laquelle réponse immunitaire protège contre l'aspergillose envahissante.

21. Utilisation selon la revendication 20, **caractérisée en ce que** la séquence identifiée no 1 contient au moins un échange d'acide aminé sélectionné parmi le groupe de
C au lieu de Y en position 43 de la séquence identifiée no 1,
L au lieu de W en position 122 de la séquence identifiée no 1,
I ou K au lieu de V en position 124 de la séquence identifiée no 1,
H au lieu de Q en position 197 de la séquence identifiée no 1,
et I au lieu de R en position 203 de la séquence identifiée no 1.

22. Utilisation selon la revendication 20 ou 21, **caractérisée en ce que** l'animal présente un système immunitaire déficient.

23. Utilisation selon la revendication 22, **caractérisée en ce que** la déficience du système immunitaire englobe l'épuisement des neutrophiles.
